# EUROPEAN PATENT APPLICATION

(11) **EP 4 226 928 A1**
(43) Date of publication of application: **16.08.2023**
(21) Application number: 21861012.9
(22) Date of filing: 12.07.2021
(51) Int. Cl.: A61K 33/30, A61K 9/10, A61K 9/12, A61K 9/72, A61K 31/185, A61P 11/00, A61P 31/00, A61P 31/12, A61K 31/191, A61P 31/14, A61P 31/16

(54) **INFECTION PREVENTION METHOD AND INFECTION PREVENTION DEVICE**

(30) Priority: 28.08.2020 JP 2020144649
(71) Applicant: Shigadry With Earth CO., LTD., Shiga 522-0222 (JP)
(72) Inventor: TANAKA Hidehiko, Hikone City, Shiga 5220222 (JP)
(74) Representative: Gille Hrabal Partnerschaftsgesellschaft mbB Patentanwälte
(86) International application number: PCT/JP2021/026156
(87) International publication number: WO 2022/044582

(57) **Abstract**

Problem to be solved

A method and device are provided for effectively preventing infections caused by viruses and bacteria that infect the respiratory system.

Solution to the problem

A method of infection prevention includes the following: a step of spraying in mist form an infection-preventive agent that contains an aqueous solution of organic acid and zinc oxide nanoparticles dispersed in the aqueous solution of organic acid, the infection-preventive agent containing 5000 to 10000 ppm of zinc salt of organic acid, and a step of causing a subject to inhale the infection-preventive agent through at least one of a nose and mouth of the subject, so as to reduce infectivity of an infectious pathogen that has entered a respiratory system of the subject.

## Description

### TECHNICAL FIELD

The present invention relates to a method of infection prevention, and more particularly, to a method and device for preventing respiratory infections caused by viruses and bacteria.

### BACKGROUND ART

Respiratory infections caused by viruses, bacteria, or fungi are transmitted by inhaling airborne viruses or bacteria, or by touching the mouth or nose with hands contaminated with viruses or bacteria.

Masks are used to prevent such respiratory infections. However, infection cannot be reliably prevented unless masks capable of completely eliminating viruses, such as N95 masks, are used in an appropriate manner.

The inventor of the present invention has found out that a medicine containing 5000 to 10000 ppm of zinc salt of organic acid has the ability to inactivate avian influenza virus and have suggested the use of this medicine as a virus inactivator (Patent Literature 1). The inventor has also disclosed that the medicine containing this zinc salt of organic acid has an antimicrobial effect not only on viruses but also on various types of bacteria and fungi (Patent Literature 2).

### Citation List

### Patent Literature

PTL 1: Japanese Patent Publication No. 2010-143875
PTL 2: Japanese Patent Publication No. 2015-113299

### SUMMARY OF THE INVENTION

### Technical Problem

During the 2020 COVID-19 pandemic, there were many clusters of infection in medical settings and many infections of medical staff who had taken preventing measures such as masks. Therefore, there is a need for a method of infection prevention that is more effective than a mask.

The purpose of this invention is to provide a method and device for infection prevention that is more effective than masks.

### Solution to Problem

To solve the above-mentioned problems, a method of infection prevention according to the present invention includes the following: a step of spraying in mist form an infection-preventive agent that contains an aqueous solution of organic acid and zinc oxide nanoparticles dispersed in the solution, the infection-preventive agent containing 5000 to 10000 ppm of zinc salt of organic acid; and a step of causing a subject to inhale the infection-preventive agent through the nose and/or mouth of the subject, so as to reduce infectivity of an infectious pathogen that has entered the respiratory system of the subject.

Examples of the organic acids in the above aqueous solution of organic acid include gluconic acid, acetic acid, citric acid, tartaric acid, malic acid, lactic acid, γ-hydroxybutyric acid, and fumaric acid. Preferred among them are organic acids that partially lactonize when dissolved in water such as γ-hydroxybutyric acid and gluconic acid.

Here, the action of the invention is explained using gluconic acid as an example. When gluconic acid is dissolved in water, part of the gluconic acid is converted to gluconolactone as shown in Chemical Formula 1. In this case, gluconic acid and gluconolactone are defined to be in equilibrium. The ratio of the gluconolactone to the gluconic acid differs depending on temperature, concentration, or pH of the aqueous solution. When acidic, the solution contains a large amount of acid, so that the gluconolactone accounts for a large proportion. Meanwhile, when the solution is alkaline, the gluconic acid is converted to salt and stabilized, so that gluconolactone accounts for a small proportion. Two components in equilibrium are as if two water tanks that are connected through a tube; when water is poured into the left-side tank, some of the water flows to the right-side tank through the tube to strike a balance.

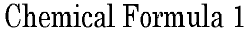

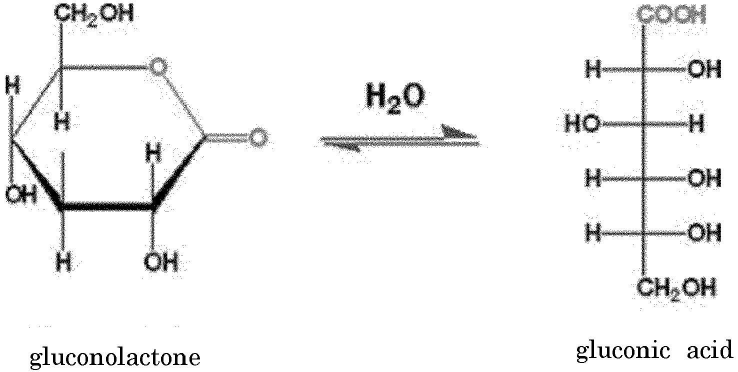

Synergistic effects produced by the concurrent use of zinc oxide nanoparticles and gluconic acid (an organic acid that partially lactonizes when dissolved in water) will now be explained by describing a case where gluconic acid comes into contact with avian influenza virus, which is a protein. As shown in Chemical Formula 2 below, any protein contains acidic and basic amino acids. When dissolved in water, the acidic amino acid, which has -COOH, is converted to negative ions (COO⁻). Meanwhile, the basic amino acid, which has -NH₂, is converted to positive ions (-NH₃⁺).

When the positive ions outnumber the negative ions or vice versa in a protein, the protein is dissolved in water. In contrast, when the positive and negative ions are equal in number (they are at the isoelectric point), the protein becomes electrically neutral without charges and remains undissolved in water. When viruses composed of proteins are at its isoelectric point, the viruses become insoluble in water and aggregate with each other. This seems to be how the viruses are inactivated.

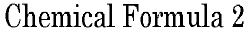

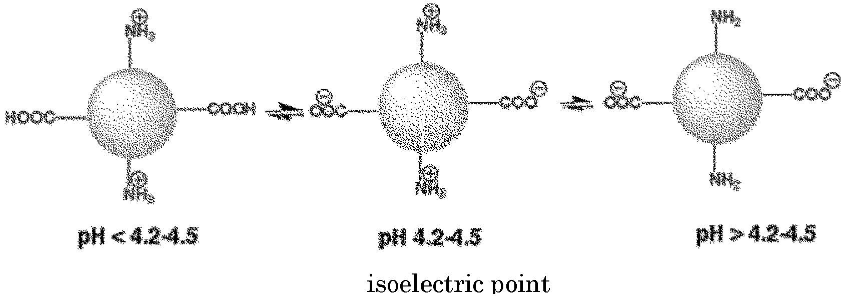

According to the present invention, zinc oxide nanoparticles react with gluconic acid (an organic acid that partially lactonizes when dissolved in water) to be converted to zinc gluconate. This zinc gluconate is considered to adjust the isoelectric point of the proteins to make the viruses aggregate and disrupt virus envelopes. This seems to be how the viruses are inactivated. Thus, the present invention in case of using an aqueous solution of zinc gluconate is particularly suitable for the prevention of infections by viruses with envelopes.

Among metal ions, mercury (Hg) is said to have the highest bactericidal effect, followed by Ag, Cu, Zn, Fe, and TiO₂ in that order. Although zinc ions are considered less bactericidal than silver ions, when an aqueous solution of zinc gluconate consisting of zinc ions and organic acid ions is used as in the present invention, the synergistic action of zinc ions and organic acid ions provides antibacterial and bactericidal activities that are not inferior to those of silver.

The action of the aqueous solution of zinc gluconate according to the present invention against viruses and bacteria is exerted not only in the air and on the surface of objects, but also inside the respiratory system. Zinc gluconate is a substance that can be added to cosmetics, breast milk substitutes, and health-promoting foods, and there is very little risk of harm to the human body if it is sprayed into the environment or ingested into the body.

When the aqueous solution of zinc gluconate or of zinc salt of other organic acids of the present invention is sprayed in mist form and inhaled through the nose and/or mouth, the mist is taken deep into the respiratory system (the area from the oral or nasal cavity to the lungs). The absorbed mist acts to inactivate viruses that either have already entered or will later enter the respiratory system. The mist further exerts a bactericidal and antimicrobial effect on bacteria and fungi.

In addition, if an aqueous solution of zinc salt of organic acid in mist form is inhaled beforehand through the nose and/or mouth, it will have an infection prevention effect for a certain period of time against viruses and bacteria that may later be taken into the respiratory system.

The zinc salt of organic acid has a high antiviral activity when its content in the solution is 5000 to 10000 ppm. In terms of antiviral activity, the zinc salt of organic acid content is preferably 6000 to 9000 ppm and more preferably 6500 to 8500 ppm. Thus, the concentration of the zinc salt of organic acid such as zinc gluconate contained in the aqueous solution of zinc salt of organic acid should be 5000 to 10000 ppm, preferably 6000 to 9000 ppm, and more preferably 6500 to 8500 ppm.

Here, the mist of an aqueous solution of zinc salt of organic acid can be sprayed into the space where the subject is present. In this case, the mist is highly effective in infection prevention because it acts on viruses and other pathogens that have already entered the respiratory system of the subject, in addition to reducing the amount of viruses and other pathogens present in the sp ace.

The mist of the aqueous solution of zinc salt of organic acid can be produced by a sprayer. Examples of such sprayers include powered or manual sprayers with a pressurized pump and spray gun, and portable sprayers such as nasal sprayers, throat sprayers, and inhalers. The sprayers allow the sprayed drug to be inhaled from near the nose and/or mouth, thus requiring a minimum amount of drug solution.

Sprayed substance (mist) having a large particle size stays in the nasal and oral cavities and rarely penetrates deep into the respiratory system. As the particle size decreases, they reach deeper into the respiratory system, such as the bronchi and lungs. As they become even smaller, they go deeper into the respiratory system, such as the lungs, but do not stay there and are expelled into the outer space by breathing. Therefore, the particle size of the mist of the solution should be small enough to allow the appropriate amount of zinc salt of organic acid to enter deep in the respiratory system and to remain there without being immediately discharged to the outside.

At least 30%, more preferably at least 50%, and even more preferably at least 70% of the particles of the mist should have a size of 0.8 to 30 µm. The particle diameters are defined as the particle size at a distance of 15 cm from the spray port of the spraying devices (e.g., sprayer, humidifier). The particle size can be measured using a laser diffraction analyzer or a laser-scattered light analyzer.

The aqueous solution of organic acid should preferably contain 40 to 50% by weight of organic acid that partially lactonizes when dissolved in water. Examples of such organic acids include gluconic acid and γ-hydroxybutyric acid. Examples of other organic acids include acetic acid, fumaric acid, citric acid, tartaric acid, malic acid, and lactic acid. The addition of fumaric acid, malic acid, or citric acid, along with an organic acid that partially lactonizes when dissolved in water, can further increase the virus inactivation and antibacterial properties.

The zinc oxide nanoparticles in the aqueous solution of zinc salt of organic acid should preferably contain nanoparticles with a diameter of 50 to 70 nm. The ultrafine particles of zinc oxide will be well dispersed in the organic acid and exhibit antimicrobial properties that are not inferior to those of silver.

Note that adding 1,3-dimethyl-2-imidazolidine to the aqueous solution of zinc gluconate can improve the solubility of the zinc gluconate in water. The 1,3-dimethyl-2-imidazolidine is preferably used because of its nontoxicity.

To solve the above-mentioned problems, the device for infection prevention according to the present invention includes an infection-preventive agent that contains an aqueous solution of organic acid and zinc oxide nanoparticles dispersed in the solution, the infection-preventive agent containing 5000 to 10000 ppm of zinc salt of organic acid; and a sprayer configured to spray the infection-preventive agent into the nasal and/or oral cavity of a subject.

The form of the sprayer in the above configuration is not particularly limited. It may be of a type that sprays mist into a space, such as a commercial sprayer or humidifier, or it may be of a type that sprays mist into the body through a nose or mouth, such as a nasal or mouth spray.

Examples of viruses that infect the respiratory system include influenza virus, RS virus, parainfluenza virus, adenovirus, rhinovirus, coronavirus, and human metapneumovirus. Examples of bacteria that infect the respiratory system include Mycoplasma, pneumoniae, Klebsiella pneumoniae, Staphylococcus aureus, Streptococcus pneumoniae, Mycobacterium tuberculosis, Chlamydophila pneumoniae, Streptococcus pyogenes, Pseudomonas aeruginosa, Haemophilus influenzae, Moraxella catarrhalis.

Of the above viruses, influenza virus, RS virus, parainfluenza virus, coronaviruses (e.g., human coronavirus, SARS-CoV, SARS-CoV-2), human metapneumoviruses have envelopes, and the effect of the present invention is considered to be particularly significant for these viruses.

The infection-preventive agent preferably has a pH of 3.0 to 6.0. In such a pH range, the agent ensures the safety of the subject when it enters its lungs, and the safety of furniture, electric appliances, etc. present in the indoor space, and also provides a greater infection prevention effect. The pH of the infection-preventive agent is more preferably 3.2 to 5.2, and even more preferably 3.4 to 4.4.

### ADVANTAGEOUS EFFECTS OF INVENTION

According to the method of infection prevention of present invention, the infection-preventive agent containing an aqueous solution of zinc salt of organic acid penetrates deep into the respiratory system of a person or animal, and inactivates viruses or bacteria that have entered the respiratory system. This reduces the infectivity of infectious pathogens, thereby preventing respiratory system infections in humans and animals.

### DESCRIPTION OF EMBODIMENT

The method of infection prevention according to the present invention includes the following: a step of spraying in mist form an infection-preventive agent that contains an aqueous solution of organic acid and zinc oxide nanoparticles dispersed in the solution, the infection-preventive agent containing 5000 to 10000 ppm of zinc salt of organic acid; and a step of causing a subject to inhale the infection-preventive agent through the nose and/or mouth of the subject, so as to reduce infectivity of an infectious pathogen that has entered the respiratory system of the subject.

As such organic acid, gluconic acid, acetic acid, citric acid, tartaric acid, malic acid, and lactic acid may be used. Preferably, organic acids that partially lactonize when dissolved in water (such as γ-hydroxybutyric acid and gluconic acid) may be used.

The infection-preventive agent can be manufactured, for example, as follows. Zinc oxide nanoparticles having a diameter of 50 to 70 nm are dispersed and stirred into an aqueous solution of gluconic acid containing 40 to 50 wt% of gluconic acid at room temperature so that the resulting zinc gluconate has a concentration of 5000 to 10000 ppm. Throughout the dispersion, the solution temperature is prevented from exceeding room temperature. It has been confirmed that while the zinc oxide nanoparticles are being dispersed and stirred in the aqueous solution of gluconic acid, the solution generates heat and undergoes gelation if heated too high.

The method of spraying is not particularly limited; commercially available sprayers, humidifiers, etc. may be used. Spray containers used for commercial nasal sprays, throat sprays, inhalants, etc. may also be used.

According to the present invention, zinc oxide nanoparticles react with gluconic acid (an organic acid that partially lactonizes when dissolved in water) to form zinc gluconate, which successfully adjusts the isoelectric point and also destroys the virus envelope, rendering it inactive. For this reason, the present invention is particularly suitable for the treatment of infections of viruses with envelopes. It also has antimicrobial activity against various bacteria and fungi.

When the infection-preventive agent in mist form is inhaled through the nose or mouth, such action is also exerted on the nasal, oral, and bronchial mucosa, and other mucous membranes. Therefore, viruses that have been taken into the respiratory system can be inactivated inside the respiratory system to reduce their infectivity, thereby preventing infection.

### Preparation of the Infection-Preventive Agent

Six different samples of an infection-preventive agent were prepared as follows, which contained different concentrations of zinc gluconate: 30,000 ppm; 10,000 ppm; 7,500 ppm; 5,000 ppm; 3,750 ppm; and 2,000 ppm. Zinc oxide nanoparticles having a diameter of 50 to 70 nm were dispersed and stirred into an aqueous solution at room temperature while the solution temperature was prevented from exceeding room temperature. The aqueous solution contained 50% of organic acid (gluconic acid in the embodiment) that partially lactonizes when dissolved in water. The resulting infection-preventive agents had a pH of 3.6.

The zinc oxide in the form of ultra-fine particles is considered to be uniformly dispersed and dissolved in water with the assistance of the organic acid (gluconic acid).

The process of dispersing the zinc oxide nanoparticles into the aqueous solution of organic acid is preferably performed while the solution is prevented from temperature rise.

### Inactivation Test of Avian Influenza Virus

Next, 0.5 ml of each sample of the aqueous solution of zinc gluconate (sterile distilled water was used for comparison) and 0.5 ml of avian influenza virus (A/whistling swan/Shimane/499/83(H5N3)10^{7.5}EID₅₀/0.1 ml) were mixed in a vortex mixer and reacted for 10 minutes at room temperature (20°C). The mixture solution of each sample and the virus was diluted ten times with sterile phosphate-buffered saline (PBS) containing an antibiotic. Then, 0.1 ml of the diluted mixture solution was inoculated into the chorioallantoic cavity of each of three 10-day-old chicken embryos. After the embryonated eggs were incubated for two days at 37°C, a hemagglutination test was conducted to check whether the virus grew in the chorioallantoic cavities. Then, the virus infectivity titers were calculated by the Reed and Muench method. The results are shown in Table 1 below.

**Table 1**

| | | Virus Infectivity Titer (log EID₅₀/0.1 ml) |
|---|---|---|
| Zinc gluconate | 30,000 ppm | 5.75 |
| Zinc gluconate | 10,000 ppm | 2.5 |
| Zinc gluconate | 7,500 ppm | 2.25 |
| Zinc gluconate | 5,000 ppm | 3.5 |
| Zinc gluconate | 3,750 ppm | 4.5 |
| Zinc gluconate | 2,000 ppm | 6.25 |
| Sterile distilled water | | 7.75 |

As apparent from Table 1, the virus infectivity titer was the lowest (2.25) when the gluconic acid concentration was 7,500 ppm. The reason for the lowest value is unknown.

The same reduction in infectivity titer as described above was observed for other viruses with respect to the infection-preventive agent with a zinc gluconate concentration of 7,500 ppm. The tested viruses are human influenza viruses and avian influenza viruses with different anti-HN types (H1N1, H1N1 subtype, and H7N7). In addition, the agent with a zinc gluconate concentration of 7,500 ppm showed a 94% reduction in infectivity titer in 3 minutes against feline caliciviruses, although the rates of reduction in infectivity titer were smaller than those of influenza viruses. Feline caliciviruses do not have an envelope.

### Growth inhibition test of various bacteria and fungi

The agent containing 7,500 ppm of zinc gluconate prepared above was diluted as appropriate, and the minimum growth inhibition concentrations against various bacteria were measured in accordance with the Minimum Inhibitory Concentration Assay I (Society of International sustaining growth for Antimicrobial Articles). The results are shown in Table 2 below.

**Table 2**

| Types of Bacteria | Minimum Inhibitory Concentration (ppm) |
|---|---|
| Staphylococcus aureus (NBRC12732) | 200 |
| E. coli (NBRC3972) | 200 |
| E. coli 0157 (ATCC43888) | 1600 |
| Salmonella enterica (NBRC3313) | 400 |
| Klebsiella pneumoniae (ATCC4352) | 400 |
| methicillin-resistant Staphylococcus aureus (IID1677) | 200 |
| Pseudomonas aeruginosa(NBRC3080) | 1600 |

The above results show that zinc gluconate at low concentrations can prevent the growth of various bacteria (Gram-positive and Gram-negative bacteria). Note that Staphylococcus aureus and Klebsiella pneumoniae can cause pneumonia symptoms.

### Antibacterial test of various bacteria and fungi

The above-mentioned infection-preventive agent containing 7,500 ppm of zinc gluconate was diluted to 2.7 times (2778 ppm zinc gluconate). It was added to various microorganism cultures to reach 200,000 Colony Forming Unit (CFU)/sample volume (ml) or more. The action time until the concentration became less than 10 CFU/sample volume (ml) was measured. For comparison, the same test was conducted using sterile saline and ethanol for disinfection. As a result, both the infection-preventive agent of the present embodiment and the disinfectant ethanol were able to reduce the concentration to less than 10 CFU/ml in 15 seconds for each of E. coli (NBRC3972), enterohemorrhagic E. coli (0157), Salmonella enterica, Vibrio parahaemolyticus, Pseudomonas aeruginosa, Klebsiella pneumoniae, Serratia marcescens, Acinetobacter, Staphylococcus aureus (NBRC12732), methicillin-resistant Staphylococcus aureus (IID1677), Staphylococcus epidermidis, Bacillus subtilis (spores), vancomycin-resistant enterococci and Streptococcus pyogenes. For Candida and black fungus, both the infection-preventive agent of the present embodiment and the ethanol for disinfection were able to reduce the concentration to less than 10 CFU/ml in 30 seconds. On the other hand, when sterile saline was used, no antibacterial effect was observed in any of the samples.

The above results show that zinc gluconate at low concentrations can inhibit the growth of various bacteria (Gram-positive and Gram-negative bacteria) and fungi.

### Coronavirus inactivation test

First, 0.9 ml of 7500 ppm of aqueous solution of zinc gluconate (phosphate buffered saline (PBS) was used for comparison) used as a sample solution and 0.1 ml of feline coronavirus solution (Feline Enteric Coronavirus, WSU 79-1683, 1.3 × 10⁵ EID₅₀/0.1 ml) were mixed in a vortex mixer and reacted at room temperature (20°C) for 30 minutes. The mixture solution of the sample and the virus was diluted to 100 times with a 5-times diluted Soybean-Casein Digest Broth with Lecithin & Polysorbate 80 (SCDLP) to stop the reaction and obtain a sample for measuring virus infection values.

Felis catus whole fetus cells used for testing were pre-seeded in 96-well plates and cultured in a CO₂ incubator for 4 days. After this, the culture supernatant was removed and replaced with Dulbecco's Modified Eagle's medium containing 1% fetal bovine serum (available from Nissui Pharmaceutical Co., Ltd.).

The wells from which the culture medium had been removed were inoculated with 25 pl of the above sample for measuring virus infection values or with a 10-fold dilution of this in PBS, and the virus was allowed to infect the cells at 37°C for 1 hour. After 1 hour, the virus solution was removed and 0.1 ml of Dulbecco's Modified Eagle's medium containing 0.2% fetal bovine serum (available from Sigma-Aldrich) was added to each well, and the cells were incubated in a CO₂ incubator for 4 days. After incubation, the cytopathic effect caused by virus growth was observed under a microscope, and the virus infection titer was calculated by the Reed and Muench method. The results of the PBS used for comparison showed that the titer was 1.3 × 10⁵ TCID₅₀/ml in 0 min, and 1.5 × 10⁵ TCID₅₀/ml in 30 min. On the other hand, in the present invention, the viral infection titer was 1.2 × 10³ TCID₅₀/ml in 30 minutes, indicating that the titer was reduced to 1/100 or less in 30 minutes.

### Test of action inside the respiratory system

Guinea pigs are known to have virus receptors in their throats and other parts of the body that are similar to those of humans. Therefore, we investigated the infection prevention action inside the respiratory system using guinea pigs.

As shown in Table 3 below, the effectiveness in preventing viral infections was investigated with and without nasal spraying of zinc gluconate and with and without spatial spraying of zinc gluconate at various times. Ten guinea pigs were used in each of the Experimental Examples.

### Nasal spray treatment

The above aqueous solution of zinc gluconate was placed in a commercially available nasal spray container, and 0.1 ml of each of the solution was sprayed on the guinea pigs so as to cover the area extending from their nasal cavity to the back. At least 50% of the particles of the mist sprayed from this spray container had a size of 0.8 to 20 µm. The pretreatment shown in Table 3 below indicates that the above treatments were given to guinea pigs that had not yet exposed to the virus, and the posttreatment indicates that the above treatments were given to guinea pigs that had just exposed to the virus.

### Spatial spraying

Guinea pigs were placed in a container that was being sprayed with the aqueous solution of zinc gluconate described above and were left in the container for 20 minutes. A commercially available sprayer was used to spray the solution at a rate of 3 ml/min. At least 55% of the particles of the mist sprayed from this sprayer had a size of 8 to 20 µm. The pretreatment shown in Table 3 below indicates that the above treatments were given to guinea pigs that had not yet exposed to the virus, and the posttreatment indicates that the above treatments were given to guinea pigs that had just exposed to the virus.

### Contact with virus

First, 10 ml of a solution of influenza A virus (Kumamoto/Y5/67(H2/N2, 10⁷ TCID₅₀/0.1 ml) was sprayed using a commercial spray container into unused containers. Next, ten guinea pigs were placed in the unused containers and were left for 20 minutes.

### Simultaneous treatment of virus and mist of zinc gluconate

Using the same spray container as above, 10 ml of the solution of influenza A virus (Kumamoto/Y5/67(H2/N2, 10⁷ TCID₅₀/0.1 ml) was sprayed into a container that was continuously being sprayed with the above aqueous solution of zinc gluconate. Ten guinea pigs were placed in this space and were left for 20 minutes while the solution was continuously sprayed. A commercially available sprayer was used to spray the solution at a rate of 3 ml/min. At least 55% of the particles of the mist sprayed from this sprayer had a size of 0.8 to 20 µm.

After the posttreatment or after the virus contact (simultaneous or continuous), each guinea pig was isolated and kept in a room temperature environment for 3 days while being fed with commercial feed and water. After this, the presence or absence of virus infection was checked by a blood test. The results are shown in Table 3 below.

**Table 3**

| | Pretreatment with mist of zinc gluconate | Posttreatment with mist of zinc gluconate | Simultaneous treatment with virus and mist of zinc gluconate | Infection rate |
|---|---|---|---|---|
| Experimental Example 1 | Nasal spray | - | - | 3/10 |
| Experimental Example 2 | Spatial spraying | - | - | 4/10 |
| Experimental Example 3 | - | Nasal spray | - | 1/10 |
| Experimental Example 4 | - | Spatial spraying | - | 3/10 |
| Experimental Example 5 | - | - | - | 8/10 |
| Experimental Example 6 | - | - | Simultaneous treatment | 1/10 |

As mentioned above, zinc gluconate has the action of inactivating viruses. This action is also exerted in living organisms such as the respiratory system. Therefore, when zinc gluconate is sprayed and inhaled through the nose and/or mouth, viruses that have been taken into the respiratory system (the area from the oral or nasal cavity to the lungs) are inactivated by the sprayed agent. For this reason, the infection rates in Experimental Examples 1-4 and 6 are significantly lower than in Experimental Example 5.

Furthermore, in Experimental Example 6, the virus present in the space is inactivated at the same time. Therefore, the infection rate is the lowest of all the Experimental Example 1-6.

Comparison of Experimental Examples 1-4 also indicates that spraying of zinc gluconate can be preceded or followed by exposure to the viral environment.

Thus, when a mist of infection-preventive agent is inhaled through the nose or mouth to exert its antiviral action on the nasal, oral, and bronchial mucosae, the amount of virus inside the respiratory system itself can be reduced. When the amount of virus inside the respiratory system is low, the possibility of viral infection is decreased. Therefore, the method of infection prevention in the present invention is highly effective in preventing viral infection.

Note that no guinea pigs have been found to exhibit any other adverse health effects from inhalation of the mist of the zinc gluconate.

In the above-mentioned Examples and Experimental Examples, gluconic acid was used as an organic acid; however, the present invention is not limited to the use of gluconic acid.

It should be understood that the above Examples have been described as examples of the implementation of the present invention. The scope of the present invention is shown not by the above description but by the scope of the claims, including all modifications and equivalents.

### INDUSTRIAL APPLICABILITY

The method of infection prevention in the present invention can safely inactivate or kill viruses and bacteria taken into the respiratory system, and has great industrial potential.

## Claims

1. A method of infection prevention, the method comprising:
spraying in mist form an infection-preventive agent that contains an aqueous solution of organic acid and zinc oxide nanoparticles dispersed in the aqueous solution of organic acid, the infection-preventive agent containing 5000 to 10000 ppm of zinc salt of organic acid, and
causing a subject to inhale the infection-preventive agent through at least one of a nose and mouth of the subject, so as to reduce infectivity of an infectious pathogen that has entered a respiratory system of the subject.

2. The method according to claim 1, wherein the aqueous solution of organic acid is prepared by dissolving an organic acid in water, the organic acid partially lactonizing when the organic acid is dissolved in water.

3. The method according to claim 1 or 2, wherein in the step of spraying the infection-preventive agent in the mist form, the infection-preventive agent is sprayed either into the at least one of the nose and mouth of the subject or into a space in which the subject is present.

4. The method according to any one of claims 1 to 3, wherein at least 30% of all particles of the infection-preventive agent sprayed in mist form have a particle size of 0.8 to 30 µm.

5. The method according to any one of claim 1 to 4, wherein the infectious pathogen is a virus with an envelope.

6. The method according to any one of claim 1 to 5, wherein the infection-preventive agent has a pH of 3.0 to 6.0.

7. An infection preventive device comprising:
an infection-preventive agent that contains an aqueous solution of organic acid and zinc oxide nanoparticles dispersed in the aqueous solution of organic acid, the infection-preventive agent containing 5000 to 10000 ppm of zinc salt of organic acid; and
a sprayer configured to spray the infection-preventive agent into at least one of a nasal cavity and oral cavity of a subject, so as to reduce infectivity of an infectious pathogen that has entered a respiratory system of the subject.

8. The device according to claim 7, wherein the sprayer is capable of controlling a particle size of the infection-preventive agent such that at least 30% of all particles of the infection-preventive agent have a particle size of 0.8 to 30 µm.

9. The device according to claim 7 or 8, wherein the aqueous solution of organic acid is prepared by dissolving an organic acid in water, the organic acid partially lactonizing when the organic acid is dissolved in water.

10. The device according to any one of claim 7 to 9, wherein the infection-preventive agent has a pH of 3.0 to 6.0.
